# EUROPEAN PATENT SPECIFICATION

(11) **EP 3 777 848 B1**
(45) Date of publication and mention of the grant of the patent: **13.03.2024**
(21) Application number: 19781046.8
(22) Date of filing: 01.04.2019
(51) Int. Cl.: A61K 31/165, A23L 33/10, A61P 9/00, A61P 9/10, A61P 9/12

(54) **COMPOSITION FOR PREVENTION OR TREATMENT OF STROKE**
ZUSAMMENSETZUNG ZUR PRÄVENTION ODER BEHANDLUNG VON SCHLAGANFALL
COMPOSITION POUR LA PRÉVENTION OU LE TRAITEMENT D'UN ACCIDENT VASCULAIRE CÉRÉBRAL

(30) Priority: 04.04.2018 KR 20180039368
(43) Date of publication of application: 17.02.2021
(73) Proprietor: Neurobiogen Co., Ltd., Seongnam-si Gyeonggi-do 13201 (KR)
(72) Inventor: PARK, Ki Duk, Seoul 02792 (KR); LEE, Changjoon, Seoul 02792 (KR); OH, Soo-Jin, Seoul 02792 (KR); PAE, Ae Nim, Seoul 02792 (KR); LIM, Sang Min, Seoul 02792 (KR); PARK, Jong Hyun, Seoul 02792 (KR); NAM, Min-Ho, Seoul 02792 (KR); KIM, Hyoung Ihl, Gwangju, Jeollanam-do 61005 (KR); PARK, Ji Young, Gwangju Jeollanam-do 61005 (KR); CHO, Jongwook, Gwangju, Jeollanam-do 61005 (KR)
(74) Representative: Cabinet Chaillot
(86) International application number: PCT/KR2019/003794
(87) International publication number: WO 2019/194494

(56) References cited:
- WO-A1-2016/052928
- KR-A- 20060 010 745
- KR-A- 20090 018 817
- KR-A- 20090 018 817
- KR-A- 20160 039 817
- US-A1- 2014 235 580
- US-A1- 2016 137 592
- US-A1- 2017 273 968

## Description

### [Technical Field]

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

The present invention relates to a pharmaceutical composition for use in a method for treating a stroke and to a food or feed composition for use in a method for alleviating a stroke, each containing (S)-2-(((4'-trifluoromethylbiphenyl-4-yl)methyl)amino)propanamide methanesulfonate.

### [Background Art]

Stroke is a cerebrovascular disease, and is one of the three major causes of death along with heart disease and cancer, and is the disease that most commonly affects the prognosis of life expectancy and increase in the population's age as the frequency thereof increases gradually. Hypertension, hyperlipidemia, atherosclerosis, and hypercholesterolemia are known to be important risk factors for cerebral stroke.

Strokes are broadly divided into two types of stroke, namely ischemic strokes caused by ischemia of brain tissue due to reduction or blockage of blood supply to brain tissue, and hemorrhagic strokes causing bleeding into brain tissue due to bursting of blood vessels. Ischemic strokes account for about 80% of all stroke cases. The mortality rate from strokes is increasing every year in foreign countries as well as in Korea, and these days there is a great deal of interest in developing drugs to treat strokes.

### [Prior art Document]

### [Patent Document]

(Patent Document 1) Korean Patent No. 10-1795562
(Patent Document 2) Korean Patent No. 10-1781060
(Patent Document 3) US 2017/0273968 relates to methods of rehabilitation of neurological disorders, including neurological deficits associated with neurotraumas such as stroke and traumatic brain injury, and the use in such methods of MAO-B inhibitors.

### [Disclosure]

### [Technical Problem]

The present inventors found that the compound (S)-2-(((4'-trifluoromethylbiphenyl-4-yl)methyl)amino)propanamide methanesulfonate of the present invention has effects of restoring deteriorated motor function caused by stroke and reducing the size of infarction in an internal capsule and is thus useful for the prevention and treatment of strokes. Based on this finding, the present invention has been completed.

### [Technical Solution]

In accordance with one aspect of the present invention, the above and other objects can be accomplished by the provision of a pharmaceutical composition for use in a method for treating strokes, containing a compound represented by the following Formula 1 or a pharmaceutically acceptable salt thereof as an active ingredient, and wherein the compound or pharmaceutically acceptable salt thereof is administered to a subject in a dosage range of 1 to 1,000 mg/kg.

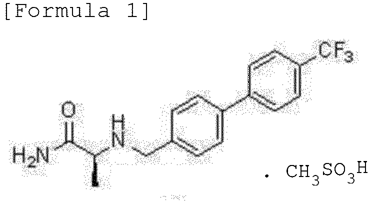

In another aspect of the present invention, provided is a food or feed composition for use in a method for alleviating strokes containing the compound as an active ingredient.

### [Advantageous Effects]

The composition containing the compound of Formula 1 of the present invention has excellent effects in preventing, alleviating and treating strokes by restoring deterioration in motor functions caused by a stroke and reducing the size of an infarction in an internal capsule.

### [Description of Drawings]

FIG. 1 is a schematic diagram illustrating a rodent stroke model;

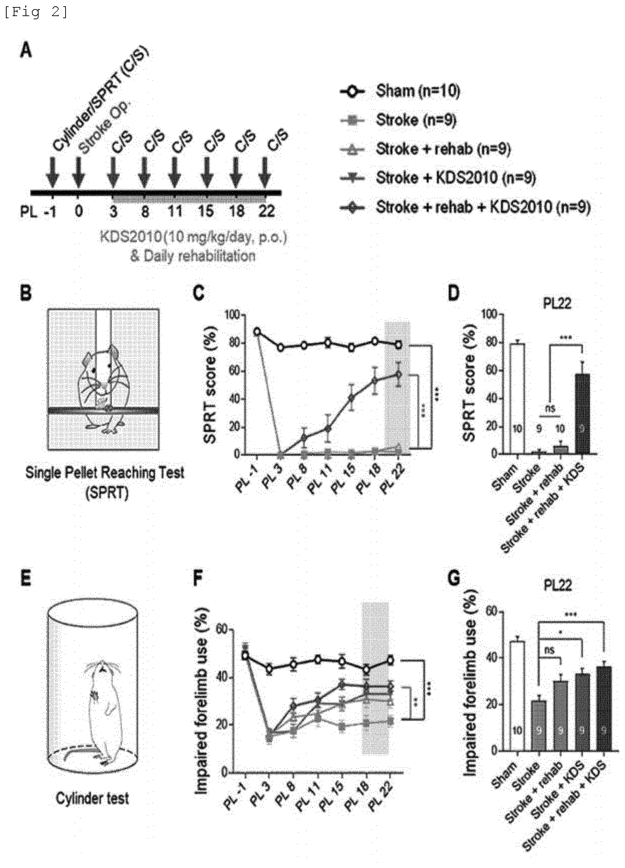

FIG. 2 shows an effect of recovering motor functions in a rodent stroke model through administration of KDS2010, more particularly, FIG. 2A is a diagram showing the total experimental period and the period of administration of the drug, FIG. 2B is an image showing a single-pellet reaching task (SPRT) test, FIGS. 2C and 2D show SPRT scores, FIG. 2E shows a cylinder test, and FIGS. 2F and 2G show impaired forelimb use (%) and opposite forelimb use during cycles in total in the cylinder test (Sham: normal control group, Stroke: stroke model, Stroke+rehab: rehabilitation-trained stroke model, Stroke+KDS2010: drug-administered stroke model, Stroke+rehab+KDS2010: drug-administered and rehabilitation-trained stroke model); and
FIG. 3 shows an effect of administration of KDS2010 on recovery of injured brain tissue in the rodent stroke model.

### [Best Mode]

Hereinafter, the present invention will be described in detail. Meanwhile, each description and embodiment disclosed in the present invention can be applied to other descriptions and embodiments. That is, all combinations of various elements disclosed in the present invention fall within the scope of the present invention. In addition, the scope of the present invention is not limited by the specific description given below.

In one aspect to accomplish the object of the present invention, the present invention is directed to a pharmaceutical composition for use in a method for treating a stroke, comprising a compound represented by the following Formula 1 or a pharmaceutically acceptable salt thereof as an active ingredient:

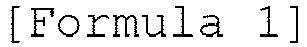

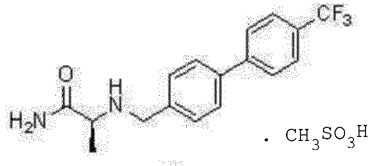

wherein the compound or pharmaceutically acceptable salt thereof is administered to a subject in a dosage range of 1 to 1,000 mg/kg.

In the present invention, the compound represented by Formula 1 is (S)-2-(((4'-trifluoromethyl biphenyl-4-yl)methyl)amino)propanamide methanesulfonate. In the present invention, the compound may be referred to as "KDS2010".

Although the study of the specific pharmacological activity of the compound is still underway, to date it has not been revealed whether or not the compound has direct effects of preventing and treating strokes.

In the present invention, there is no particular limitation as to the method for obtaining (S)-2-(((4'-trifluoromethyl biphenyl-4-yl)methyl)amino)propanamide methanesulfonate, and the compound can be chemically synthesized by a method known in the art, or may be selected from commercially available substances.

The (S)-2-(((4'-trifluoromethyl biphenyl-4-yl)methyl)amino)propanamide methanesulfonate of the present invention may be present in a solvated form or an unsolvated form. The (S)-2-(((4'-trifluoromethyl biphenyl-4-yl)methyl)amino)propanamide methanesulfonate of the present invention may be present in a crystalline or amorphous form, and all of these physical forms fall within the scope of the present invention.

The pharmaceutical composition of the present invention may include not only the compound represented by Formula 1 but also a pharmaceutically acceptable salt thereof. As used herein, the term "pharmaceutically acceptable salt" means a salt of the compound in combination with another substance, and refers to a substance capable of exhibiting pharmacologically similar activity.

The types of the pharmaceutically acceptable salt include, but are not limited to, inorganic acid salts such as hydrochloride, hydrobromide, phosphate or sulfate, and organic acid salts such as carboxylate or sulfonate. In addition, the type of carboxylate includes, but is not limited to, acetate, maleate, fumarate, malate, citrate, tartrate, lactate or benzoate. Further, the type of sulfonate includes, but is not limited to, methanesulfonate, ethanesulfonate, benzenesulfonate, toluene sulfonate or naphthalene disulfonate.

As used herein, the term "stroke" means a symptom of a local neurological defect that is suddenly caused by a cerebral blood flow abnormality. Such a stroke may include a cerebral infarction, wherein the death of brain cells is caused by blockage in cerebral blood vessels due to blood clots or the like, or brain hemorrhage caused by bursts of cerebral blood vessels. Specific symptoms of stroke include sudden headache and vomiting, hemiparalysis or paralysis of body parts, sensory paralysis and loss of body parts, language disorders (dysphasia or pronunciation disorder), facial nerve disorders, ataxia and the like. The stroke in the present invention may mean paralysis.

The pharmaceutical composition of the present invention can exert effects of restoring deterioration in motor functions caused by stroke and reducing the size of the infarction in an internal capsule.

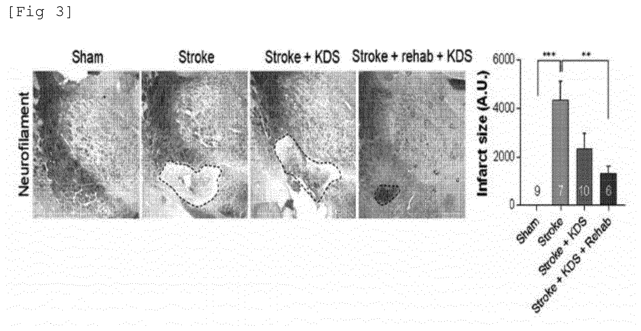

In a specific embodiment of the present invention, the result of treatment of an animal stroke model with the compound of Formula 1 of the present invention shows that motor function recovery was facilitated and the size of the infarction in an internal capsule was reduced, compared to an animal stroke model not administered with the drug (FIGS. 2 and 3).

The result demonstrated that the composition containing the compound of Formula 1 of the present invention has an excellent effect of preventing or treating strokes.

As used herein, the term "preventing" or "prevention" refers to any action that inhibits or delays the onset of a stroke through administration of the composition containing the compound of Formula 1 of the present invention. As used herein, the term "treatment" refers to any action which alleviates or beneficially alters the symptoms of the disease through administration of the composition containing the compound of Formula 1 of the present invention.

The pharmaceutical dosage form of the composition for preventing or treating strokes of the present invention may be a pharmaceutically acceptable salt thereof, and the composition may be used alone or bonded to or suitably combined with another pharmaceutically active compound.

The composition for preventing or treating stroke of the present invention may further include a pharmaceutically acceptable carrier.

The composition for preventing or treating strokes of the present invention can be prepared into a pharmaceutical formulation using a method well known in the art to provide rapid, sustained or delayed release of the active ingredient after administration to a mammal. In the preparation of the formulation, preferably, the active ingredient is mixed or diluted with a carrier or encapsulated into a carrier in the form of a container.

Accordingly, the composition for preventing or treating stroke of the present invention is used as an oral formulation, such as a powder, granule, tablet, capsule, suspension, emulsion, syrup or aerosol, or a formulation such as an external preparation, suppository or sterile injectable solution, according to a conventional method. The composition may further include a suitable carrier, excipient and diluent commonly used in the preparation of compositions.

For example, the carrier that can be included in the composition of the present invention includes, but is not limited to, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil and the like. The preparation is carried out using an ordinary diluent or excipient such as a filler, extender, binder, wetting agent, disintegrating agent or surfactant.

Solid formulations for oral administration include tablets, pills, powders, granules, capsules and the like, and the solid formulation is prepared by mixing at least one excipient such as starch, calcium carbonate, sucrose, lactose or gelatin. In addition, lubricants such as magnesium stearate and talc may also be used, in addition to simple excipients.

Liquid formulations for oral administration include suspensions, liquids/solutions, emulsions, syrups and the like. In addition to commonly used simple diluents, water and liquid paraffin, various excipients, such as wetting agents, sweeteners, fragrances, and preservatives, may be also included.

Formulations for parenteral administration include sterile aqueous solutions, non-aqueous solvents, suspensions, emulsions, lyophilized preparations and suppositories. Non-aqueous solvents and suspensions may include propylene glycol, polyethylene glycol, vegetable oils such as olive oil, and injectable esters such as ethyl oleate. As a base for suppositories, Witepsol, macrogol, tween 61, cacao butter, laurin butter and glycerogelatin may be used.

The pharmaceutical composition of the present invention may be administered as a single therapeutic agent or in combination with another therapeutic agent, and may be administered sequentially or simultaneously with a conventional therapeutic agent. The pharmaceutical composition may be administered once or several times. Considering all of the factors described above, it is important to administer the composition in an amount capable of obtaining the maximum effect in a minimum amount without causing side effects, and such an amount can be easily determined by those skilled in the art.

As used herein, the term "administration" refers to introduction of the pharmaceutical composition of the present invention into a patient by any suitable method, and the route of administration of the composition of the present invention may be any one of a variety of oral or parenteral routes enabling the composition to reach the target tissue.

The method of administration of the pharmaceutical composition according to the present invention is not particularly limited, and may be any method commonly used in the art. As a non-limiting example of the administration method, the composition may be administered by an oral or parenteral administration method. The pharmaceutical composition according to the present invention can be prepared into various formulations according to a desired administration method.

The frequency of administration of the composition of the present invention is not particularly limited, but may be once a day or several times a day in a portionwise manner.

A daily dosage of the composition containing the compound of Formula 1 of the present invention as an active ingredient is 1 to 1,000 mg/kg, preferably 11 to 100 mg/kg, and the composition may be administered once or several times in a portionwise manner.

In another aspect, the present invention is directed to a food or feed composition for use in a method for alleviating strokes containing the compound represented by Formula 1 as an active ingredient.

The food composition for preventing or alleviating strokes of the present invention includes formulations such as pills, powders, granules, acupuncture agents, tablets, capsules, or liquids, and the foods to which the composition of the present invention can be added include, for example, various foods, such as beverages, gum, teas, vitamin complexes and health supplement foods.

The food composition for preventing or alleviating stroke of the present invention may include, as an essential ingredient, the composition or an active component thereof or a physiologically acceptable salt thereof, and there is no particular limitation as to other ingredients. Similar to common foods, the food composition may further include additional ingredients such as various herbal extracts, food supplement additives or natural carbohydrates.

As mentioned above, food supplement additives may also be added, and the food supplement additives include food additives commonly used in the art, for example, flavoring agents, aromas, coloring agents, fillers, stabilizers and the like.

Examples of the natural carbohydrates include conventional sugars including monosaccharides such as glucose and fructose, disaccharides such as maltose and sucrose, polysaccharides such as dextrin and cyclodextrin, and sugar alcohols such as xylitol, sorbitol and erythritol. In addition to the ingredients described above, natural flavoring agents (for example, rebaudioside A, glycyrrhizin or the like) and synthetic flavoring agents (saccharin, aspartame or the like) can be advantageously used as flavoring agents.

In addition to the ingredients described above, the food composition for preventing or alleviating strokes of the present invention may include a variety of nutrients, vitamins, minerals (electrolytes), flavoring agents such as synthetic flavoring agents and natural flavoring agents, coloring agents and fillers (such as cheese or chocolate), pectic acid and salts thereof, alginic acid and salts thereof, organic acids, protective colloidal thickeners, pH-adjusting agents, stabilizers, preservatives, glycerin, alcohol, carbonizing agents used in carbonated beverages, and the like. In addition, the food composition may include natural fruit juice and fruit flesh for the production of fruit juice beverages and vegetable beverages. These ingredients can be used alone or in combination.

In the present invention, the health supplement food includes a health functional food, a health food, and the like.

The term "health functional food" has the same meaning as food for special health use (FoSHU), and means a food having excellent pharmaceutical and medicinal effects, which is processed to efficiently provide the functions of bioregulation and nutrition supply. Here, "function" (or "functional") means obtaining effects useful for health applications, such as nutrient control or physiological actions on the structures and functions of the human body. The food of the present invention can be produced by a method commonly used in the art, and can be produced by adding raw materials and ingredients conventionally added in the art. In addition, any formulation of the food may also be used without limitation, as long as it is acceptable as food. The food composition of the present invention can be prepared into various types of formulations and has the advantages of being free from side effects that may occur upon long-term administration of the drug because it contains food as a raw material, unlike general drugs. In addition, owing to excellent portability thereof, the food according to the present invention can be taken as a supplement to improve the effects of preventing or alleviating strokes.

The feed composition of the present invention may mean any suitable natural or artificial diet, single meal, or component thereof that is eaten, taken or digested by an animal, and may be prepared in various forms known in the art.

The type of the feed is not particularly limited, and a feed commonly used in the art may be used. Non-limiting examples of the feed include vegetable feed such as grains, nuts, food processing byproducts, algae, fiber, pharmaceutical byproducts, fats and oils, starches, gourds or grain byproducts; and animal feed such as proteins, inorganic substances, fats and oils, minerals, fats and oils, single-cell proteins, zooplankton and food. These ingredients may be used alone or in combinations of two or more thereof.

For administration, in addition to the compound described above, the feed additive composition of the present invention may include a combination of one or more of organic acids such as citric acid, fumaric acid, adipic acid, and lactic acid, phosphates such as potassium phosphate, sodium phosphate and polymerized phosphate, and natural antioxidants such as polyphenols, catechin, tocopherol, vitamin C, green tea extracts, chitosan and tannic acid, and may optionally include other conventional additives such as buffers and bacteriostatic agents. In addition, the composition may be prepared by further adding diluents, dispersants, surfactants, binders and lubricants.

The feed composition can be easily administered in the state of being mixed directly with animal feed or combined with other ingredients. The dosage varies within a wide range depending on factors such as the animal's weight, health conditions, diet, method of administration and severity of disease.

In another aspect, the present invention is directed to a method for preventing or treating strokes including administering the compound represented by Formula 1 to a subject other than a human.

The method for preventing or treating strokes may further include rehabilitation training.

As used herein, the term "subject" may mean any animal, including a human, who has or is likely to develop a stroke. The animal may be a human or a mammal, such as a cow, horse, sheep, pig, goat, camel, antelope, dog or cat, which requires treatment of similar symptoms, but is not limited thereto.

The method for preventing or treating strokes according to the present invention may specifically include administering a pharmaceutically effective amount of the composition to a subject who has or is likely to develop a stroke.

### [Best model

Hereinafter, the configurations and effects of the present invention will be described in more detail with reference to examples. However, it will be obvious to those skilled in the art that these examples are provided only for illustration of the present invention and should not be construed as limiting the scope of the present invention.

### Example 1: Test method

### 1-1. Preparation of drug to be administered

A target drug was prepared to determine the therapeutic effect thereof on strokes. The drug to be administered was prepared as described in Korean Patent Registration No. 10-1746060. Specifically, (S)-2-(((4'-trifluoromethylbiphenyl-4-yl)methyl)amino)propanamide methanesulfonate, disclosed in Example 9 in accordance with the synthesis method in the Patent gazette (hereinafter, referred to as "KDS2010"), was synthesized.

### 1-2. Production of rodent stroke model

Light stimuli was applied to the posterior limb of the internal capsule of a 9 week-old SD rat to produce a white-matter cerebral infarction animal model.

Specifically, the animal was anesthetized with an anesthetic drug, a combination of ketamine (ketamine hydrochloride, 100 mg/kg) and xylazine (7 mg/kg), and a hole was drilled at a position of 2.0 mm rearwards and 3.1 mm laterally outwards from the skull bregma using stereotaxis, and an optical fiber (inner diameter of 62.5 µm, outer diameter of 125 µm) was inserted to a depth of 7.2 mm to target the posterior limb of the internal capsule. Rose bengal (20 mg/kg) was injected into the tail vein, and optical stimulation was conducted using a 3.7 mW green laser for 1.5 minutes through an optical fiber. Then, the optical fiber was removed and the operation was completed, ketoprofen (2 mg/kg) was injected for pain control, and then the animal was allowed to recover. The control group was injected with 0.9% saline, instead of rose bengal.

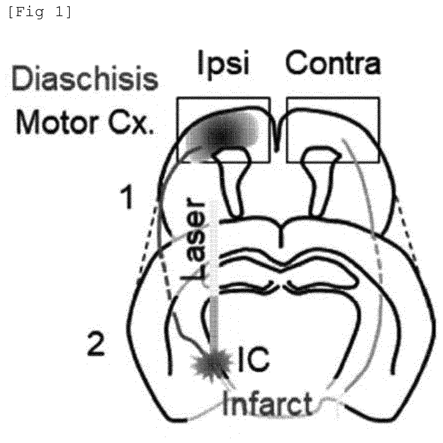

The outline of the brain structure of the produced cerebral infarction animal model is shown in FIG. 1.

### 1-3. Drug administration and rehabilitation training

Rats in the group administered with KDS2010 as a drug were orally administered with a solution of KDS2010 in saline at a concentration of 10 mg/kg once a day (FIG. 2A) .

Rats in the rehabilitation-trained group practiced a single-pellet reaching task (SPRT) for 20 minutes daily for 3 weeks after stroke surgery. Meanwhile, rats in the non-rehabilitation-trained group practiced SPRT only twice a week for three weeks.

### 1-4. Behavior test

In order to determine the effect of KDS2010 on rodent stroke models, a cylinder test was conducted to determine asymmetry of the use of the forelimb, and a single-pellet reaching task was conducted to evaluate elaborate motor skills.

For the cylinder test, animals were placed in a transparent cylinder (20 cm in diameter and 30 cm in height) to observe which limb was used more when supporting the cylinder wall with the forelimbs. A total of 20 movements were recorded, and the ratio of the use of the forelimb corresponding to the stroke and the opposite forelimb was calculated during the total number of cycles.

For the single-pellet reaching task, an acrylic box (45 cm X 13 cm X 40 cm) having a 1 cm vertical slit on the front wall thereof was used, and a sugar pellet was placed in front of the slit. Animals were prepared in a state in which they sought food by being fasted until their body weight dropped to 90% of their original body weight. The proportion of the number of pellets remaining in the mouth, without dropping, to 20 pellets in total was calculated.

### 1-5. Neurofiber staining

In order to determine the size of the infarction in an internal capsule, the rats after the behavior test were sacrificed for histological examination, and brain tissues were prepared therefrom. Nerve fibers were stained using the extracted brain tissue, a neurofiber antibody (SMI-31R, BioLegend, CA, USA) and a DAB staining kit (TL-060-QHD, Thermo, MA, USA).

### Example 2: Test results

### 2-1. Determination of effect of restoring motor functions using single-pellet reaching task and cylinder test

As can be seen from B to D of FIG. 2, the result of evaluation of the motor function through a single-pellet reaching task in the same manner as in Example 1-4 above showed that the rodent stroke model exhibited considerably impaired motor function. The rodent stroke model substantially failed to recover motor skills even though they were rehabilitated through training after induction of stroke. On the other hand, the KDS2010-drug administrated and rehabilitation-trained group recovered motor function to an almost normal level.

In addition, the result of the determination of the effect of restoring motor function using the cylinder test, as can be seen from E to G of FIG. 2, showed that the rodent stroke model had significantly impaired motor function, whereas the drug-administered group (Stroke+ KDS2010) significantly recovered motor function compared to the control group.

Therefore, the result demonstrated that administration of the KDS2010 drug to the stroke model can recover impaired motor function due to stroke.

### 2-2. Determination of effect of restoring motor function using single-pellet reaching task and cylinder test

The nerve fibers were stained in the same manner as in Example 1-5 above to observe the size of the infarctions in internal capsules.

As can be seen from FIG. 3, the result showed that the rodent stroke models significantly increased the size of the infarction in the internal capsules, whereas the drug-administered group (Stroke+KDS) had considerably larger size of the infarction in the internal capsules than that of the control group. In particular, the experimental group (Stroke+rehab+KDS), which was subjected to rehabilitation training in combination with drug administration, had further reduced infarction sizes in the internal capsules.

Therefore, the result described above showed that KDS2010 can reduce the infarction area and restore motor function, thereby being useful in the prevention or treatment of paralysis, particularly strokes.

## Claims

1. A pharmaceutical composition for use in a method for treating a stroke, comprising a compound represented by the following Formula 1 or a pharmaceutically acceptable salt thereof as an active ingredient: 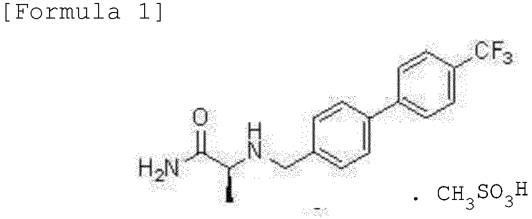 wherein the compound or pharmaceutically acceptable salt thereof is administered to a subject in a daily dosage range of 1 to 1,000 mg/kg.

2. The pharmaceutical composition for use according to claim 1, wherein the stroke is an ischemic stroke or hemorrhagic stroke.

3. The pharmaceutical composition for use according to claim 1, wherein the composition facilitates recovery of deteriorated motor function due to the stroke.

4. The pharmaceutical composition for use according to claim 1, wherein the composition reduces a size of an infarction in an internal capsule.

5. A food or feed composition for use in a method for alleviating a stroke comprising a compound represented by the following Formula 1 as an active ingredient:

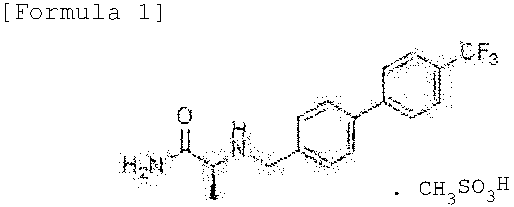

## Patentansprüche

1. - Pharmazeutische Zusammensetzung zur Verwendung in einem Verfahren zur Behandlung eines Schlaganfalls, umfassend eine Verbindung, dargestellt durch die folgende Formel 1 oder ein pharmazeutisch annehmbares Salz davon als Wirkstoff: 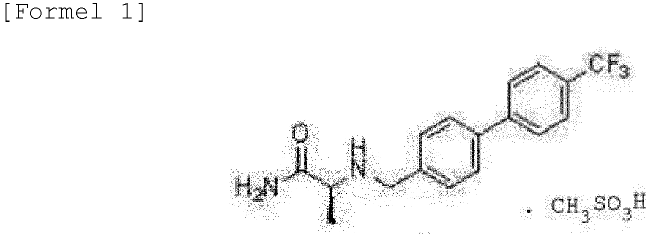 wobei die Verbindung oder das pharmazeutisch annehmbare Salz davon einem Subjekt in einer täglichen Dosis im Bereich von 1 bis 1000 mg/kg verabreicht wird.

2. - Pharmazeutische Zusammensetzung zur Verwendung nach Anspruch 1, wobei der Schlaganfall ein ischämischer Schlaganfall oder ein hämorrhagischer Schlaganfall ist.

3. - Pharmazeutische Zusammensetzung zur Verwendung nach Anspruch 1, wobei die Zusammensetzung die Wiederherstellung der beeinträchtigten Motorik aufgrund des Schlaganfalls erleichtert.

4. - Pharmazeutische Zusammensetzung zur Verwendung nach Anspruch 1, wobei die Zusammensetzung die Größe eines Infarkts in einer internen Kapsel reduziert.

5. - Lebensmittel- oder Futterzusammensetzung zur Verwendung in einem Verfahren zur Linderung eines Schlaganfalls, umfassend eine Verbindung, dargestellt durch die folgende Formel 1 als Wirkstoff:

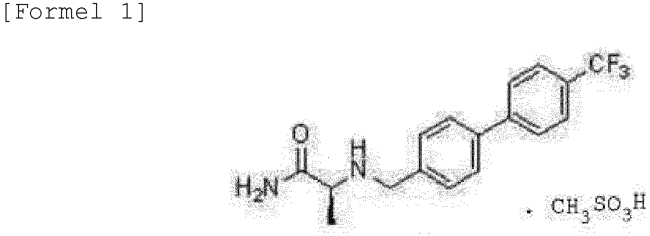

## Revendications

1. - Composition pharmaceutique pour utilisation dans une méthode de traitement d'un accident vasculaire cérébral, comprenant un composé représenté par la Formule 1 suivante ou un sel pharmaceutiquement acceptable de celui-ci en tant que principe actif : 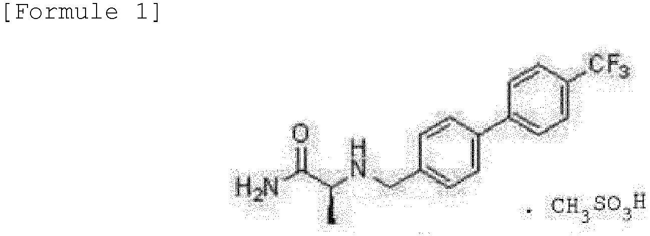 dans laquelle le composé ou le sel pharmaceutiquement acceptable de celui-ci est administré à un sujet dans une plage posologique quotidienne de 1 à 1000 mg/kg.

2. - Composition pharmaceutique pour utilisation selon la revendication 1, dans laquelle l'accident vasculaire cérébral est un accident vasculaire cérébral ischémique ou un accident vasculaire cérébral hémorragique.

3. - Composition pharmaceutique pour utilisation selon la revendication 1, dans laquelle la composition facilite la récupération d'une fonction motrice détériorée due à l'accident vasculaire cérébral.

4. - Composition pharmaceutique pour utilisation selon la revendication 1, dans laquelle la composition réduit la taille d'un infarctus dans une capsule interne.

5. - Aliment ou composition alimentaire pour utilisation dans une méthode pour soulager un accident vasculaire cérébral comprenant un composé représenté par la Formule 1 suivante en tant que principe actif :
